# EUROPÄISCHE PATENTSCHRIFT

(11) **EP 3 911 144 B1**
(45) Veröffentlichungstag und Bekanntmachung des Hinweises auf die Patenterteilung: **05.07.2023**
(21) Anmeldenummer: 20700802.0
(22) Anmeldetag: 14.01.2020
(51) Int. Cl.: A01G 7/00, G01L 19/06

(54) **DRUCKERFASSUNGSEINRICHTUNG FÜR EINE MESSVORRICHTUNG ZUM MESSEN EINES DRUCKZUSTANDSWERTS EINER PFLANZENPROBE UND VERFAHREN ZUR HERSTELLUNG EINER DRUCKERFASSUNGSEINRICHTUNG**
PRESSURE-SENSING DEVICE FOR A MEASURING INSTRUMENT FOR MEASURING A PRESSURE STATUS VALUE OF A PLANT SPECIMEN, AND METHOD FOR PRODUCING A PRESSURE-SENSING DEVICE
DISPOSITIF DE DÉTECTION DE PRESSION POUR UN DISPOSITIF DE MESURE DESTINÉ À MESURER UNE VALEUR DE L'ÉTAT DE PRESSION D'UN ÉCHANTILLON VÉGÉTAL ET PROCÉDÉ DE FABRICATION D'UN DISPOSITIF DE DÉTECTION DE PRESSION

(30) Priorität: 15.01.2019 DE 102019200378
(43) Veröffentlichungstag der Anmeldung: 24.11.2021
(73) Patentinhaber: Robert Bosch GmbH, 70442 Stuttgart (DE)
(72) Erfinder: BOECKER, Matthias, 72760 Reutlingen (DE)
(86) Internationale Anmeldenummer: PCT/EP2020/050765
(87) Internationale Veröffentlichungsnummer: WO 2020/148254

(56) Entgegenhaltungen:
- EP-B1- 2 672 806

## Beschreibung

### Stand der Technik

Der hydrostatische Überdruck in pflanzlichen Zellen wird als Turgordruck bezeichnet und stellt einen Parameter zur Bewertung eines Zustands, insbesondere eines Bewässerungszustands einer Pflanze dar. Durch Erfassen des Turgordrucks bzw. eines turgordruckabhängigen Druckzustandswerts, z.B. an Blättern der Pflanze, können Rückschlüsse gezogen werden, ob die Wasserversorgung der Pflanze ausreichend oder eine Bewässerung der Pflanze erforderlich ist.

Zur Messung des Turgordrucks bzw. einer den Turgordruck repräsentierenden Größe, einer Pflanzenprobe, z.B. eines Blatts einer Pflanze, kommen zunehmend Messvorrichtungen zum Einsatz, welche auf einem nichtdestruktiven oder nicht-invasiven Messverfahren beruhen. Typischerweise wird die Pflanzenprobe hierzu mittels einer Klemmvorrichtung eingespannt und ein den Turgordruck repräsentierendes Drucksignal wird mittels einer Sensoreinrichtung aufgenommen.

Beispielsweise beschreibt die DE 10 2006 043 058 A1 eine Messvorrichtung zum Messen eines Druck-Zustandswertes einer Pflanze mit einer Druckerfassungseinrichtung, welche einen plattenförmigen Sensorträger mit einer einseitig offenen Vertiefung und einer am Boden der Vertiefung angeordneten Sensoreinrichtung aufweist, wobei die Vertiefung mit einer elastischen Druckkopplungslage aus Silikon ausgefüllt ist. Zur Messung des Druck-Zustandswertes wird die Pflanzeprobe mittels eines Klemmteils an die Druckkopplungslage der Messvorrichtung angedrückt, so dass die Sensoreinrichtung einen Druck erfassen kann. Die DE 10 2009 032 872 A1 beschreibt eine ähnliche Druckerfassungseinrichtung, wobei eine Sensoreinrichtung an den Boden einer Vertiefung eines Sensor-Klemmblocks angeklebt und in der Vertiefung verdrahtet wird. Anschließend erfolgt ein Verfüllen der Vertiefung mit einer Vergussmasse zur Ausbildung einer Druckkopplungslage. Aus der EP 2 244 548 B1 ist eine weitere Druckerfassungseinrichtung dieser Art bekannt.

Die EP 2 672 806 B1 beschreibt eine Druckerfassungseinrichtung zum Messen eines Turgordrucks einer Pflanze mit einer Sensorfassung und einer in einer Vertiefung der Sensorfassung aufgenommenen Sensoreinrichtung. Die Sensoreinrichtung weist eine Leiterplatte mit einem daran angeordneten Drucksensor auf, wobei der Sensoreinsatz in der Vertiefung in ein Polymermaterial eingegossen ist. Zur Herstellung der Messeinrichtung wird die Sensoreinrichtung in die Vertiefung der Sensorfassung eingesetzt und anschließend schichtweise mit Polymermaterial vergossen. Eine Montagefläche der Sensorvorrichtung und eine Montagefläche der Sensorfassung befinden sich auf gegenüberliegenden Seiten der Leiterplatte.

### Offenbarung der Erfindung

Die vorliegende Erfindung betrifft ein Verfahren gemäß Anspruch 1 und eine Druckerfassungseinrichtung gemäß Anspruch 6.

Gemäß einem ersten Aspekt der Erfindung ist ein Verfahren zur Herstellung einer Druckerfassungseinrichtung für eine Messvorrichtung zum Messen eines Druckzustandswerts einer Pflanzenprobe vorgesehen. Der Druckzustandswert kann durch einen Turgordruck der Pflanzenprobe oder eine vom Turgordruck abhängige Zustandsgröße gebildet sein.

Das Verfahren umfasst ein Montieren einer Sensoreinrichtung zum Erfassen des Druckzustandswerts an einem Trägersubstrat. Das Trägersubstrat kann insbesondere als eine flächige Platte realisiert sein. Die Sensoreinrichtung ist zum Erfassen eines Drucks eingerichtet und wird an einer Montageoberfläche des Trägersubstrats befestigt, z.B. an dieses angelötet oder angeklebt wird.

Nach dem Montieren der Sensoreinrichtung erfolgt ein Befestigen eines Rahmens an der Montageoberfläche des Trägersubstrats, wobei der Rahmen eine Befestigungsfläche, eine entgegengesetzt zu der Befestigungsfläche orientierte Kontaktfläche und eine Öffnung definierende und sich zwischen der Befestigungsfläche und der Kontaktfläche erstreckende Innenfläche aufweist, wobei der Rahmen derart an dem Trägersubstrat angeordnet wird, dass die Befestigungsfläche dem Trägersubstrat zugewandt ist und die Innenfläche die Sensoreinrichtung umschließt. In diesem Schritt wird der Rahmen, der eine sich zwischen einer ersten Oberfläche oder Kontaktfläche und einer entgegengesetzt zu dieser gelegenen zweiten Oberfläche oder Befestigungsfläche erstreckende Durchgangsöffnung aufweist, derart auf die Montagefläche des Trägersubstrats aufgesetzt, dass die Sensoreinirchtung in der Öffnung des Rahmens angeordnet und die Befestigungsfläche des Rahmens dem Trägersubstrat zugewandt ist.

Die Befestigungsfläche des Rahmens wird mit der Montageoberfläche des Trägersubstrats verbunden. Die Kontaktfläche dient zur Anlage an und zum Klemmen der Pflanzenprobe.

In einem weiteren Schritt erfolgt ein Füllen der Öffnung des Rahmens mit einem Füllmaterial zur Ausbildung einer elastischen Druckkopplungslage. Das Füllmaterial wird hierzu in einem visko-elastischen oder fließfähigen Zustand der Öffnung des Rahmens zugeführt und füllt diese zumindest teilweise, vorzugsweise komplett aus, so dass die in der Öffnung angeordnete Sensoreinrichtung vollständig in das Füllmaterial eingebettet ist. Das Füllmaterial ist zur Anlage an die Pflanzenprobe vorgesehen und dient zur Übertragung einer Druckkraft der Pflanzenprobe an die Sensoreinrichtung.

Gemäß einem zweiten Aspekt der Erfindung ist eine Druckerfassungseinrichtung für eine Messvorrichtung zum Messen eines Druckzustandswerts einer Pflanzenprobe vorgesehen. Die Druckerfassungseinrichtung gemäß diesem Aspekt der Erfindung kann insbesondere durch ein Verfahren gemäß dem ersten Aspekt der Erfindung hergestellt werden. Die in der vorliegenden Offenbarung beschriebenen Merkmale und Vorteile des Verfahrens gelten somit in analoger Weise auch für die Druckerfassungsvorrichtung und umgekehrt.

Die Druckerfassungsvorrichtung umfasst ein Trägersubstrat mit einer Montageoberfläche, eine an der Montageoberfläche des Trägersubstrats angeordneten Sensoreinrichtung zum Erfassen des Druckzustandswerts und einen an der Montageoberfläche des Trägersubstrats befestigten Rahmen, welcher eine dem Trägersubstrat zugewandte Befestigungsfläche, eine entgegengesetzt zu der Befestigungsfläche orientierte Kontaktfläche zur Anlage an die Pflanzenprobe und eine Öffnung definierende und sich zwischen der Befestigungsfläche und der Kontaktfläche erstreckende Innenfläche aufweist, wobei die Innenfläche die Sensoreinrichtung umschließt. Demnach ist der Rahmen als von dem Trägersubstrat separate Komponente realisiert, welche eine Durchgangsöffnung aufweist, innerhalb derer die Sensoreinrichtung angeordnet ist.

Ferner weist die Druckerfassungseinrichtung eine die Öffnung füllende elastische Druckkopplungslage zur Anlage an die Pflanzenprobe zum Übertragen einer Kraft an die Sensoreinrichtung. Die Druckkopplungslage ist aus einem elastischen Füllmaterial, wie z.B. einem Silikonmaterial oder einem Polymermaterial gebildet, welches die in der Öffnung angeordnete Sensoreinrichtung vollständig einbettet. Vorzugsweise bildet die Druckkopplungslage eine Kopplungsfläche aus, welche die Kontaktfläche des Rahmens stetig, insbesondere planar fortsetzt.

Eine der Erfindung zugrundeliegende Idee besteht darin, eine Druckerfassungseinrichtung für eine Messvorrichtung zum Messen eines turgordruckabhängigen Druckzustandswerts einer Pflanzenprobe mit einem Trägersubstrat oder einer Trägerplatte, auf welcher eine Drucksensoreinrichtung montiert wird, und einem von dem Trägersubstrat bzw. der Trägerplatte separaten Rahmen mit einer Durchgangsöffnung zu realisieren. Dadurch kann die Sensoreinrichtung zur Erfassung des Druckzustandswerts auf der Montageoberfläche des Trägersubstrats befestigt werden bevor der Rahmen angebracht wird. Dies erleichtert die Montage der Sensoreinrichtung. Ferner kann die Öffnung des Rahmens verkleinert werden bzw. die Innenfläche, welche die Öffnung definiert und die Sensoreinrichtung umgibt, kann mit minimalen Abstand zu der Sensoreneinrichtung angeordnet werden, ohne dass dies die Montage der Sensoreinrichtung erschwert. Ein kleinerer Durchmesser bzw. eine kleinere Querschnittsfläche der Öffnung verringert die zur Erzeugung eines bestimmten Drucks notwendige Kraft, wodurch eine Klemmkraft, mit welcher die Druckerfassungseinrichtung an die Pflanzenprobe gedrückt wird, verringert werden kann. Dies verringert sowohl die Gefahr, die Pflanze zu beschädigen, als auch die Gefahr, dass die Sensoreinrichtung beschädigt wird.

Vorteilhafte Ausgestaltungen und Weiterbildungen ergeben sich aus den Unteransprüchen sowie aus der Beschreibung unter Bezugnahme auf die Figuren der Zeichnung.

Gemäß einer Ausführungsform des Verfahrens ist vorgesehen, dass das Montieren der Sensoreinrichtung ein Befestigen der Sensoreinrichtung an dem Trägersubstrat und ein Kontaktieren der Sensoreinrichtung umfasst. Das Befestigen der Sensoreinrichtung kann insbesondere ein Ankleben, ein Anlöten, ein Anclipsen oder eine ähnliche stoff- oder formschlüssiges Befestigen der Sensoreinrichtung an der Montageoberfläche des Trägersubstrats umfassen. Zum Kontaktieren werden an der Sensoreinrichtung vorgesehene elektrische Anschlüsse mit an dem Trägersubstrat vorgesehenen elektrischen Anschlüssen oder Terminals elektrisch verbunden, z.B. über Draht- oder Leiterbahnstrukturen.

Gemäß einer weiteren Ausführungsform des Verfahrens wird das Füllmaterial in einem fließfähigen Zustand in die Öffnung des Rahmens eingeführt und in der Öffnung gehärtet. Das Härten des Füllmaterials kann insbesondere eine Vernetzungsreaktion umfassen, wodurch die elastischen Eigenschaften des Füllmaterials bzw. der Druckkopplungslage vorteilhaft einstellbar sind.

Gemäß einer weiteren Ausführungsform des Verfahrens wird das Füllmaterial von einer durch die Kontaktfläche des Rahmens definierten Vorderseite in die Öffnung des Rahmens eingeführt. Auf diese Weise wird die Öffnung sukzessive beginnend an der Montageoberfläche des Trägersubstrats aufgefüllt, wodurch vorteilhaft eine gleichmäßige Füllung bei Verringerung der Gefahr der Bildung von Luftblasen oder anderen Einschlüssen erreicht wird. Optional kann der Rahmen sich von der Innenfläche aus erstreckende bzw. durch diese definierte Entlüfungskanäle aufweisen, welche die Gefahr von Lufteinschlüssen weiter verringern.

Gemäß einer weiteren Ausführungsform des Verfahrens ist vorgesehen, dass der Rahmen stoffschlüssig, insbesondere durch Ankleben, oder formschlüssig, insbesondere durch eine Clip-Verbindung, an dem Trägersubstrat befestigt wird. Eine stoffschlüssige Verbindung bietet insbesondere den Vorteil, dass die Befestigungsfläche des Rahmens gegenüber dem Trägersubstrat besonders zuverlässig abgedichtet wird. Eine formschlüssige Verbindung kann beispielsweise sehr schnell und einfach montiert werden.

Gemäß einer Ausführungsform der Druckerfassungseinrichtung ist vorgesehen, dass die Innenfläche im Bereich der Kontaktfläche des Rahmens, insbesondere an der Kontaktfläche einen ersten Durchmesser bzw. eine erste Querschnittsfläche der Öffnung und im Bereich der Befestigungsfläche des Rahmens, insbesondere an der Befestigungsfläche einen zweiten Durchmesser bzw. eine zweite Querschnittsfläche der Öffnung definiert, wobei der zweite Durchmesser bzw. die zweite Querschnittsfläche größer als der erste Durchmesser bzw. die erste Querschnittsfläche der Öffnung ist. Die Öffnung verengt sich somit von dem Trägersubstrat bzw. der Befestigungsfläche zu der Kontaktfläche hin. Damit bildet die Innenfläche in Bezug auf eine senkrecht auf die Kontaktfläche stehende Achse eine Hinterschneidung aus. Auf diese Weise wird durch die Innenfläche ein Hindernis realisiert, welches einem Herausbewegen der Druckkopplungslage aus der Öffnung entgegenwirkt. Dies ist insbesondere von Vorteil, wenn die Kontaktfläche von der Pflanzenprobe entfernt wird. Somit können besonders elastische bzw. weiche Druckkopplungslagen verwendet werden, die sich sehr gut an die Pflanzenprobe anschmiegen. Dadurch wird die Genauigkeit der Messergebnisse erhöht. Ferner wird auch die Verformung der Druckkopplungslage beim Lösen von der Pflanzenprobe verringert, wodurch die Gefahr einer Beschädigung der Sensoreinrichtung reduziert wird. Ferner bietet der sich zu von der Kontaktfläche zu der Befestigungsfläche bzw. dem Trägersubstrat hin erweiternde Querschnitt bzw. Durchmesser der Öffnung den Vorteil, dass am Trägersubstrat eine große Montagefläche für die Sensoreinrichtug bereitgestellt wird. Gleichzeitig kann jedoch eine Anlagefläche der Druckkopplungslage zur Anlage an die Pflanzenprobe verringert werden, wodurch für die Erzeugung eines vorbestimmten Anlagedrucks nur eine kleine Kraft notwendig ist und des Weiteren die Druckerfassungseinrichtung auch an kleinere Blätter problemlos angebracht werden kann.

Zur Realisierung verschiedener Durchmesser bzw. Querschnittsflächen an der Kontaktfläche und der Befestigungsfläche kann sich die Innenfläche zumindest abschnittsweise angeschrägt oder gewinkelt relativ zu einer sich quer oder senkrecht zu der Kontaktfläche erstreckenden Achse erstrecken. Beispielsweise kann die Innenfläche eine kreisförmige Öffnung definieren, wobei die Innenfläche einen zumindest abschnittsweise kegelstumpfförmigen Querschnitt der Öffnung definiert. Eine solche kegelstumpfförmige Geometrie bietet den Vorteil, dass diese besonders einfach und damit kostengünstig durch zerspanende Bearbeitungsverfahren herstellbar ist. Selbstverständlich kann die Innenfläche auch dreieck-, rechteck- oder polygonförmige Öffnungsquerschnitte definieren, wobei die Innenfläche einen zumindest abschnittsweise pryramiden- oder allgemein polyederstumpfförmigen Querschnitt der Öffnung definiert.

Gemäß einer weiteren Ausführungsform der Druckerfassungseinrichtung ist vorgesehen, dass das Trägersubstrat durch eine Leiterplatte gebildet ist. Dies erleichtert insbesondere die Kontaktierung bzw. Verdrahtung der Sensoreinrichtung.

Gemäß einer weiteren Ausführungsform der Druckerfassungseinrichtung weist die Sensoreinrichtung ein Sensorelement zur Erfassung des Druckzustandswerts und zur Erzeugung eines den Turgordruck bzw. den Druckzustandswert repräsentierenden Drucksignals in Form einer MEMS-Struktur und eine Ausleseeinheit in Form eines ASIC zur Verarbeitung des vom Sensorelement erzeugbaren Drucksignals auf. "MEMS" steht hierbei als Abkürzung für den englischen Ausdruck "microelectronic and microelectromechanical system". "ASIC" steht als Abkürzung für den englischen Ausdruck "application-specific integrated circuit". Durch den ASIC kann das durch das Sensorelement bereitgestellte Drucksignal direkt in der Sensoreinrichtung digitalisiert werden. Insbesondere kann die MEMS-Struktur vorteilhaft über den ASIC kontaktiert oder verdrahtet werden. Somit wird die Montage der Sensoreinrichtung weiter vereinfacht. Bei Verwendung einer Leiterplatte als Trägersubstrat ergibt sich zudem der Vorteil, dass übliche Lötprozesse zur Verdrahtung und Befestigung der mit einer MEMS-Struktur und einem ASIC realisierten Sensoreinrichtung verwendet werden können, wodurch eine einfache und kostengünstige Montage erleichtert wird.

Gemäß einer weiteren Ausführungsform der Druckerfassungseinrichtung ist vorgesehen, dass der Rahmen ein magnetisches oder magnetisierbares Material aufweist. Beispielsweise kann der Rahmen aus einem Basisteil, welches die Kontaktfläche, die Befestigungsfläche und die die Öffnung definierende Innenfläche des Rahmens bereitstellt bzw. ausbildet, und einem Magnetteil aus einem magnetisierbaren oder magnetischen Material zusammengesetzt sein, wobei der Magnetteil in eine Aufnahmestruktur, z.B. eine Aufnahmenut des Basisteils aufgenommen ist. Das Basisteil kann beispielsweise aus einem Kunststoffmaterial hergestellt sein. Alternativ kann der Rahmen auch insgesamt aus einem magnetischen oder magnetisierbaren Material gebildet sein.

Gemäß einer weiteren Ausführungsform der Druckerfassungseinrichtung ist vorgesehen, dass der Rahmen aus einem Kunststoffmaterial gebildet ist. Hier kommen insbesondere Thermoplastmaterialien, Duroplastmaterialien oder Elastomermaterialien in Frage.

Allgemein kann der Rahmen beispielsweise einen rechteckförmigen, einen kreisförmigen, einen polygonalen oder ähnlichen Außenumfang aufweisen. Der Außenumfang des Rahmens wird hierbei durch eine entgegengesetzt zu der Innenfläche gelegenen Außenumfangsfläche, welche sich zwischen der Befestigungsfläche und der Kontaktfläche des Rahmens erstreckt, realisiert. Die Außenumfangsfläche kann sich beispielsweise parallel zu der Innenfläche erstrecken, so dass ein zu der Innenfläche

In Bezug auf Richtungsangaben und Achsen, insbesondere auf Richtungsangaben und Achsen, die den Verlauf von physischen Strukturen betreffen, wird hierin unter einem Verlauf einer Achse, einer Richtung oder einer Struktur "entlang" einer anderen Achse, Richtung oder Struktur verstanden, dass diese, insbesondere die sich in einer jeweiligen Stelle der Strukturen ergebenden Tangenten jeweils in einem Winkel von kleiner 45 Grad, bevorzugt kleiner 30 Grad und insbesondere bevorzugt parallel zueinander verlaufen.

In Bezug auf Richtungsangaben und Achsen, insbesondere auf Richtungsangaben und Achsen, die den Verlauf von physischen Strukturen betreffen, wird hierin unter einem Verlauf einer Achse, einer Richtung oder einer Struktur "quer" zu einer anderen Achse, Richtung oder Struktur verstanden, dass diese, insbesondere die sich in einer jeweiligen Stelle der Strukturen ergebenden Tangenten jeweils in einem Winkel von größer oder gleich 45 Grad, bevorzugt größer oder gleich 60 Grad und insbesondere bevorzugt senkrecht zueinander verlaufen.

Unter einer "Pflanzenprobe" wird hierin ein Bestandteil einer intakten Pflanze, insbesondere ein Pflanzenorgan bzw. Pflanzengewebe, beispielsweise ein Blatt oder ein Stiel der Pflanze verstanden. Der Bestandteil der Pflanze kann hierbei auch vom Rest der Pflanze abgetrennt sein.

Die vorliegende Erfindung wird nachfolgend anhand der in den schematischen Figuren der Zeichnungen angegebenen Ausführungsbeispiele näher erläutert. Es zeigen dabei:
- Fig. 1: eine schematische Schnittansicht einer Messvorrichtung zur Messung eines Druckzustandswerts einer Pflanzenprobe, welche eine Druckerfassungseinrichtung gemäß einem Ausführungsbeispiel der Erfindung aufweist;
- Fig. 2: eine perspektivische Ansicht einer schematischen Schnittdarstellung einer Druckerfassungseinrichtung gemäß einem Ausführungsbeispiel der Erfindung;
- Fig. 3: eine perspektivische Ansicht einer schematischen Schnittdarstellung einer Druckerfassungseinrichtung gemäß einem weiteren Ausführungsbeispiel der Erfindung;
- Fig. 4: eine perspektivische Ansicht einer schematischen Schnittdarstellung einer Druckerfassungseinrichtung gemäß einem weiteren Ausführungsbeispiel der Erfindung;
- Fig. 5: eine perspektivische Ansicht einer schematischen Schnittdarstellung einer Druckerfassungseinrichtung gemäß einem weiteren Ausführungsbeispiel der Erfindung;
- Fig. 6: eine Draufsicht auf eine Kontaktfläche eines Rahmens einer Druckerfassungseinrichtung gemäß einem weiteren Ausführungsbeispiel der Erfindung;
- Fig. 7: einen ersten Schritt eines Verfahrens zur Herstellung einer Druckerfassungseinrichtung gemäß einem Ausführungsbeispiel der Erfindung;
- Fig. 8: einen weiteren Schritt des Verfahrens gemäß einem Ausführungsbeispiel der Erfindung;
- Fig. 9: einen weiteren Schritt des Verfahrens gemäß einem Ausführungsbeispiel der Erfindung; und
- Fig. 10: einen weiteren Schritt des Verfahrens gemäß einem Ausführungsbeispiel der Erfindung.

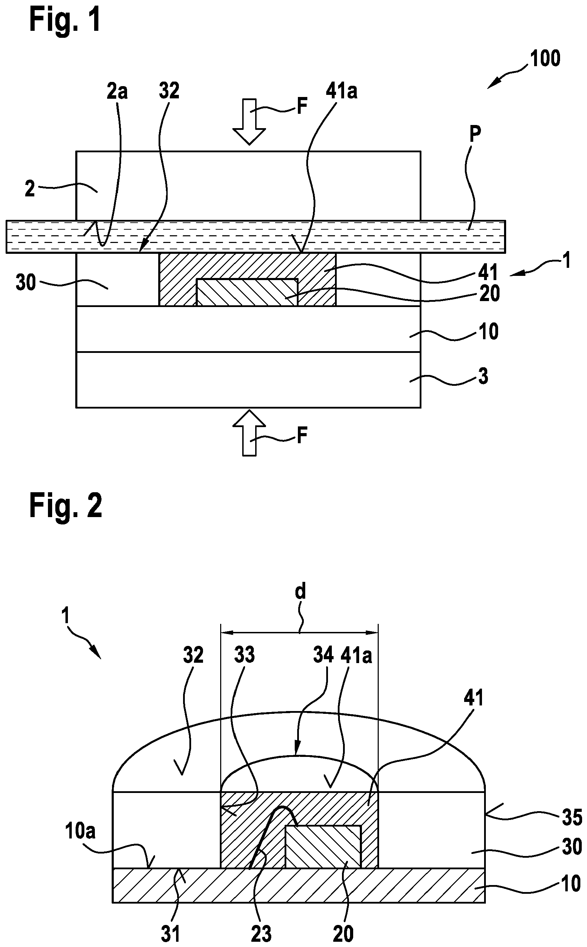

Fig. 1 zeigt schematisch eine Schnittansicht einer Messvorrichtung 100 zum Messen eines Druckzustandswerts einer Pflanzenprobe P. Der gemessene Druckzustandswert ist abhängig vom sogenannten Turgordruck der Pflanzenprobe P. Der hydrostatische Überdruck in pflanzlichen Zellen wird als Turgordruck bezeichnet und stellt einen Bewässerungszustand der Pflanze repräsentierenden Parameter dar. Typischerweise repräsentiert ein hoher Turgordruck einen hohen Wassergehalt und damit einen ausreichenden Bewässerungszustand der Pflanze, während ein niedriger Turgordruck einen geringen Wassergehalt in der Pflanze anzeigt. Die in Fig. 1 beispielhaft dargestellte Messvorrichtung 100 weist eine Druckerfassungseinrichtung 1 und eine Klemmeinrichtung 2 auf. Optional kann außerdem ein Magnet 3 vorgesehen sein.

Wie in Fig. 1 schematisch dargestellt, umfasst die Druckerfassungseinrichtung 1, welche nachfolgend noch im Detail erläutert wird, ein Trägersubstrat 10, eine Sensoreinrichtung 20, einen Rahmen 30 und eine Druckkopplungsschicht oder Druckkopplungslage 41.

Die Klemmeinrichtung 2 ist als plattenförmiges Bauteil realisiert, welches eine Klemm-Kontaktfläche 2a aufweist. Die Klemmeinrichtung 2 kann insbesondere teilweise oder ganz aus einem magnetischen oder magnetisierbaren Material gebildet sein und dient als Gegenlager zum Klemmen einer Pflanzenprobe P, wie dies in Fig. 1 beispielhaft dargestellt ist.

Zum Messen des Druckzustandswerts wird eine Pflanzenprobe P, z.B. ein Blatt einer Pflanze, zwischen der Klemm-Kontaktfläche 2a der Klemmeinrichtung 2 und einer Kontaktfläche 32 des Rahmens 30 der Druckerfassungseinrichtung 1 eingeklemmt bzw. eingespannt, wie dies in Fig. 1 schematisch dargestellt ist. Hierbei wird eine vorbestimmte Klemmkraft F auf die Pflanzenprobe ausgeübt, welche beispielsweise durch eine von dem optionalen Magneten 3 auf die Klemmeinrichtung 2 wirkende magnetische Kraft aufgebracht wird. Das Druckkopplungsmedium bzw. die Druckkopplungslage 41, welche elastisch verformbar ist, kommt dadurch mit einer Kontaktfläche 41a in Anlage an die Pflanzenprobe P und kann dadurch eine Druckkraft an die Sensoreinrichtung 20 übertragen. Die Sensoreinrichtung 20 erfasst dadurch eine den Druckzustandswert bzw. den Turgordruck repräsentierende Größe.

In den Fign. 2 bis 5 sind verschiedene Druckerfassungseinrichtungen 1 gezeigt, welche wie voranstehend beschrieben zur Erfassung des Druckzustandswerts einsetzbar sind. Wie in Fig. 2 beispielhaft dargestellt, weist die Druckerfassungseinrichtung 1 ein Trägersubstrat 10, eine Sensoreinrichtung 20, einen Rahmen 30 und eine Druckkopplungsschicht oder Druckkopplungslage 41 auf.

Wie in der perspektivischen Schnittansicht der Fig. 2 erkennbar ist, kann das Trägersubstrat 10 als Scheibe mit einem kreisförmigen Außenumfang realisiert sein. Selbstverständlich sind auch andere Umfangsgeometrien denkbar. Allgemein ist das Trägersubstrat 10 als flächiges, plattenförmiges Bauteil realisiert, welches eine Montageoberfläche 10a bereitstellt. Das Trägersubstrat 10 dient als Träger oder Halterung für die weiteren Komponenten der Druckerfassungseinrichtung. Optional kann das Trägersubstrat 10 als Leiterplatte realisiert sein, auf welcher elektrische Kontaktstellen bzw. Kontaktierungsstrukturen wie Leiterbahnen (nicht dargestellt) oder dergleichen ausgebildet sind.

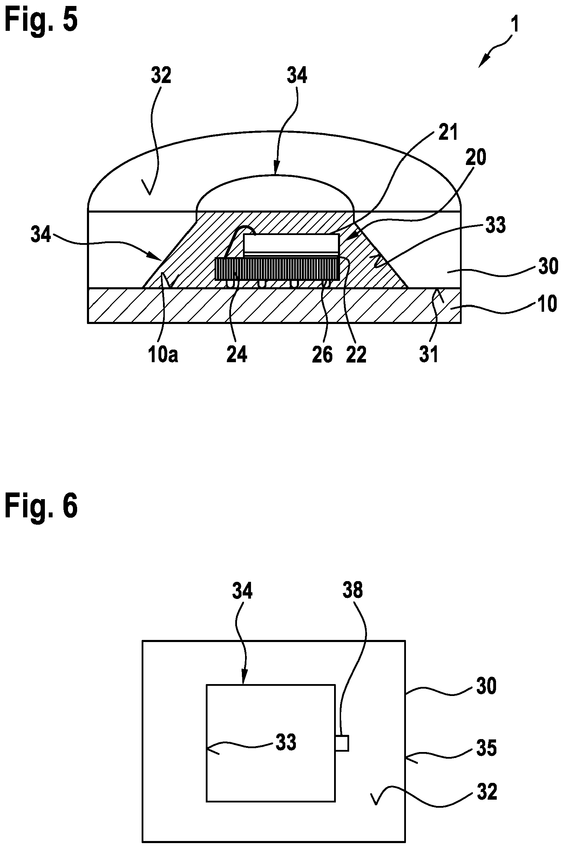

Die Sensoreinrichtung 20 ist in Fig. 2 lediglich schematisch als Block dargestellt. Die Sensoreinrichtung 20 ist allgemein als Kraft-/ Drucksensor realisiert, der zur Erfassung einer einen Druck repräsentierenden Größe eingerichtet ist. Wie in Fig. 5 schematisch dargestellt ist, kann die Sensoreinrichtung 20 ein Sensorelement 21 zur Erfassung des Druckzustandswerts und zur Erzeugung eines den Druckzustandswert bzw. den Turgordruck repräsentierenden Drucksignals aufweisen, welches als MEMS-Struktur realisiert ist. Die MEMS-Struktur kann beispielsweise einen Trägerchip mit einer daran angebrachten verformbaren Membran aufweisen, wobei auf der Membran elektrische Widerstände angebracht sind, die zu einer Wheatston-Brücke verschaltet sind. Durch eine Verformung der Membran infolge einer Druckänderung entsteht proportional zur Verformung eine elektrische Spannungsänderung, welche als ein Drucksignal repräsentative Größe dargestellt wird. Wie in Fig. 5 außerdem dargestellt ist, kann optional zusätzlich zu dem Sensorelement 21 eine Ausleseeinheit 22 in Form eines ASIC zur Verarbeitung des vom Sensorelement erzeugbaren Drucksignals vorgesehen sein. Wie in Fig. 5 weiterhin dargestellt ist, können die Ausleseeinheit 22 und das Sensorelement 21 auf einer gemeinsamen Sensorleiterplatte 24 angeordnet und kontaktiert sein. Alternativ hierzu können auch Drucksensoren anderer Bauart verwendet werden, deren Drucksignal an einer externen Ausleseeinheit weiterverarbeitet bzw. ausgewertet wird.

Wie in Fig. 2 schematisch dargestellt, ist die Sensoreinrichtung 20 an der Montageoberfläche 10a des Trägersubstrats 10 befestigt. Beispielsweise kann die Sensoreinrichtung 20 an der Montageoberfläche 10a angeklebt oder angelötet sein. Wie in Fig. 2 außerdem schematisch dargestellt ist, ist die Sensoreinrichtung 20 über eine Verdrahtung 23 elektrisch kontaktiert, z.B. mit an dem Trägersubstrat 10 vorgesehenen Anschlussstrukturen (nicht dargestellt). Wie in Fig. 5 beispielhaft dargestellt ist, kann eine Kontaktierung und Befestigung der Sensorleiterplatte 24 durch Lötverbindungen 26 gleichzeitig realisiert werden.

Wie in Fig. 2 ferner dargestellt ist, kann der Rahmen 30 als kreisringförmiges Bauteil realisiert sein. Fig. 6 zeigt hingegen schematisch eine Draufsicht auf einen Rahmen 30, welcher mit einem rechteckförmigen Außenumfang und einer rechteckförmigen Öffnung 34 realisiert ist. Allgemein weist der Rahmen 30 eine Befestigungsfläche 31, eine entgegengesetzt zu der Befestigungsfläche 31 orientierte Kontaktfläche 32, eine Öffnung 34 definierende und sich zwischen der Befestigungsfläche 31 und der Kontaktfläche 32 erstreckende Innenfläche 33 und eine entgegengesetzt zu der Innenfläche 33 orientierte und sich zwischen der Befestigungsfläche 31 und der Kontaktfläche 32 erstreckende Außenumfangsfläche 35 auf.

Die Befestigungsfläche 31 und die Kontaktfläche 32 können insbesondere planar sein und erstrecken sich vorzugsweise parallel zueinander, wie dies in Fig. 2 schematisch dargestellt ist. Die Kontaktfläche 32 dient zur Anlage an die Pflanzenprobe P, wie dies anhand der Fig. 1 erläutert wurde. Die Befestigungsfläche 31 des Rahmens 30 dient zur Verbindung bzw. Befestigung des Rahmens 30 an der Montageoberfläche 10a des Trägersubstrats 10. Die Außenumfangsfläche 35 verbindet die Befestigungsfläche 31 mit der Kontaktfläche 32 und definiert dadurch die Umfangsgeometrie des Rahmens 30. Wie in den Fign. 2 bis 5 dargestellt ist, kann die Außenumfangsfläche 35 beispielsweise einen kreisförmigen Außenumfang des Rahmens 30 definieren. In Fig. 6 ist beispielhaft ein durch die Außenumfangsfläche 35 definierter rechteckiger Außenumfang des Rahmens 30 dargestellt. Selbstverständlich sind auch andere Umfangsgeometrien des Rahmens 30 denkbar, z.B. ein ovaler, ein dreickförmiger, ein trapezförmiger oder anderer polygonaler Außenumfang. In den Fign. 2 bis 6 ist beispielhaft dargestellt, dass sich die Innenfläche 33 und die Außenumfangsfläche 35 parallel zueinander erstrecken. Es ist selbstverständlich auch denkbar, dass die Innenfläche 33 und die Außenumfangsfläche 35 unterschiedliche Geometrien der Öffnung 34 und des Außenumfangs definieren.

Wie in Fig. 2 ferner dargestellt, verbindet die Innenfläche 33 die Befestigungsfläche 31 mit der Kontaktfläche 32 und bildet dadurch eine sich zwischen der Befestigungsfläche 31 und der Kontaktfläche 32 erstreckende Durchgangsöffnung 34 des Rahmens 30 aus. Die Innenfläche 33 definiert ferner die Querschnittsform der Öffnung 34. Beispielsweise kann die Öffnung 34 einen kreisförmigen Querschnitt aufweisen, z.B. einen zylinderförmigen Querschnitt wie dies in Fig. 2 schematisch dargestellt ist. Selbstverständlich sind auch andere Querschnittsformen denkbar, wie z.B. ein rechteckförmiger Querschnitt, wie dies in Fig. 6 beispielhaft dargestellt ist. Andere polygonale Querschnittsformen sind ebenfalls denkbar.

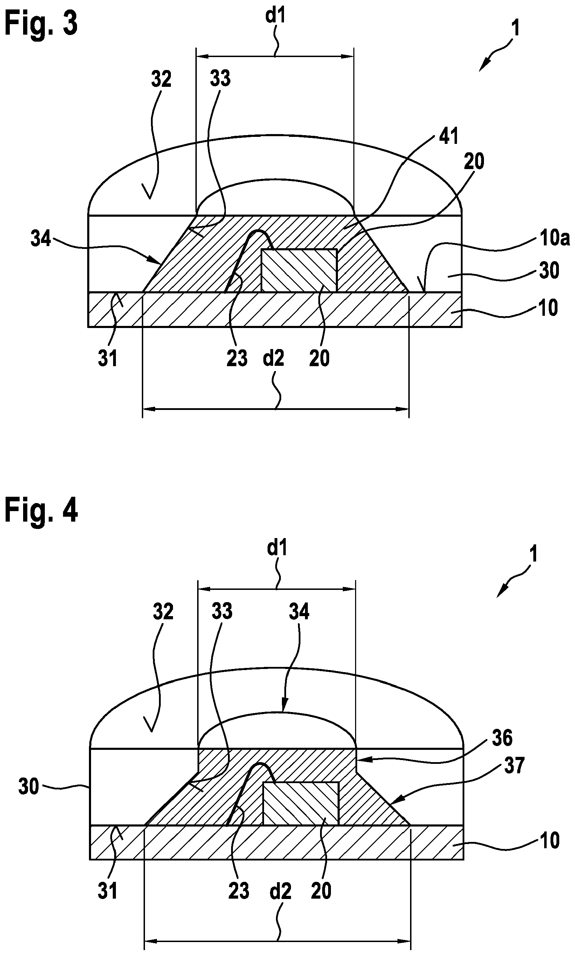

Fig. 2 zeigt beispielhaft einen Rahmen 30 dessen Innenfläche 33 einen konstanten Querschnitt bzw. Durchmesser d der Öffnung 34 definiert. Wie in den Fign. 3 bis 5 beispielhaft dargestellt ist, kann auch vorgesehen sein, dass ein von der Innenfläche 33 definierter Durchmesser bzw. eine von der Innenfläche 33 definierte Querschnittsfläche der Öffnung 34 von der Kontaktfläche 32 zu der Befestigungsfläche 31 hin zunimmt.

Fig. 3 zeigt beispielhaft, dass die Innenfläche 33 einen kegelstumpfförmigen Querschnitt der Öffnung definiert, wobei ein erster Durchmesser d1 bzw. eine erste Querschnittsfläche der Öffnung 34 an der Kontaktfläche 32 kleiner ist als ein zweiter Durchmesser d2 bzw. eine zweite Querschnittsfläche der Öffnung 34 an der Befestigungsfläche 31.

In den Fign. 4 und 5 ist beispielhaft ein teilweise kegelstumpfförmiger Querschnitt der Öffnung 34 dargestellt. Insbesondere definiert die Innenfläche 33 im Bereich der Kontaktfläche 32 einen zylindrischen Querschnittsbereich 36 der Öffnung 34 mit einem ersten Durchmesser d1 und einen sich zwischen diesem zylindrischen Bereich 36 und der Befestigungsfläche 31 erstreckenden kegelstumpfförmigen Bereich 37, wobei die Öffnung 34 an der Befestigungsfläche 32 einen zweiten Durchmesser d2 aufweist, der größer als der erste Durchmesser d1 ist.

Neben den in den Fign. 3 bis 5 beispielhaft dargestellten Querschnittsverläufen sind auch andere Verläufe denkbar, z.B. ein pyramidenstumpfförmiger oder zumindest abschnittsweise pyramidenstufmpfförmiger Verlauf. Allgemein definiert die Innenfläche 33 des Rahmens 30 im Bereich der Kontaktfläche 32 des Rahmens 30 einen ersten Durchmesser d1 bzw. eine erste Querschnittsfläche der Öffnung 34 und im Bereich der Befestigungsfläche 31 des Rahmens 30 einen zweiten Durchmesser d2 bzw. eine zweite Querschnittsfläche der Öffnung 34, wobei der zweite Durchmesser d2 bzw. die zweite Querschnittsfläche größer als der erste Durchmesser d1 bzw. die erste Querschnittsfläche der Öffnung 34 ist.

Der Rahmen 30 kann ein magnetisches oder magnetisierbares Material aufweisen oder aus einem solchen gebildet sein. In diesem Fall kann vorteilhaft auf den Magnet 3 verzichtet werden, wenn die Klemmeinrichtung 2 der Messeinrichtung 100 als Magnet realisiert ist. Der Rahmen 30 kann auch aus einem Kunststoffmaterial, z.B. aus einem Thermoplastmaterial, einem Duroplastmaterial oder Elastomer gebildet sein. Hierbei ist auch denkbar, dass in das Kunststoffmaterial magnetische oder magnetisierbare Materialbereiche eingebracht sind.

Der Rahmen 30 ist so an dem Trägersubtrat 10 befestigt, dass die Befestigungsfläche 31 der Montageoberfläche 10a des Trägersubstrats 10 zugewandt ist und die Sensoreinrichtung 20 in der Öffnung 34 des Rahmens 30 angeordnet ist. Damit umschließt die Innenfläche 33 die Sensoreinrichtung 20, wie dies in den Fign. 2 bis 5 schematisch dargestellt ist. Der Rahmen 30 kann beispielsweise mit dem Trägersubstrat 10 verklebt sein oder formschlüssig an diesem befestigt sein, z.B. durch eine Clip-Verbindung.

Die Druckkopplungslage 41 ist in der Öffnung 34 des Rahmens 30 angeordnet. Insbesondere kann die Öffnung 34 vollständig durch die Druckkopplungslage 41 ausgefüllt sein, wie dies in den Fign. 2 bis 5 beispielhaft dargestellt ist. Wie in den Fign. 2 bis 5 erkennbar ist, kann vorgesehen sein, dass ein durch die Druckkopplungslage 41 gebildete Kontaktfläche 41a, welche von dem Trägersubstrat 10 abgewandt gelegen und zur Anlage an die Pflanzenprobe P vorgesehen ist, sich planar zu der Kontaktfläche 31 des Rahmens 30 erstreckt. Die Druckkopplungslage 41 ist aus einem elastisch verformbaren Füllmaterial 40 gebildet, z.B. einem Silkonmaterial oder einem Polymermaterial, und dient zum Übertragen einer Kraft an die Sensoreinrichtung 20, wie dies voranstehend im Zusammenhang mit Fig. 1 erläutert wurde.

Wie in den Fign. 3 bis 5 erkennbar ist, kann durch den sich von der Kontaktfläche 32 des Rahmens 30 zu der Befestigungsfläche 31 hin vergrößernden Durchmesser bzw. Querschnittsfläche der Öffnung 34 an der Montagefläche 10a des Trägersubstrats 10 ausreichend Platz für die Sensoreinrichtung bereitgestellt werden, während die Öffnung 34 an der Kontaktfläche 31 einen kleinen Durchmesser d1 aufweist. Zur Erzeugung eines bestimmten Drucks an der Kontaktfläche 41a der Druckkopplungslage 41, deren Größe durch den ersten Durchmesser d1 der Öffnung 34 festgelegt ist, ist dadurch vorteilhaft nur eine geringe Kraft nötig. Ferner wird die Druckkopplungslage 41 durch den in Richtung der Kontaktfläche 31 kleiner werdenden Durchmesser der Öffnung 41 formschlüssig in der Öffnung 41 gehalten.

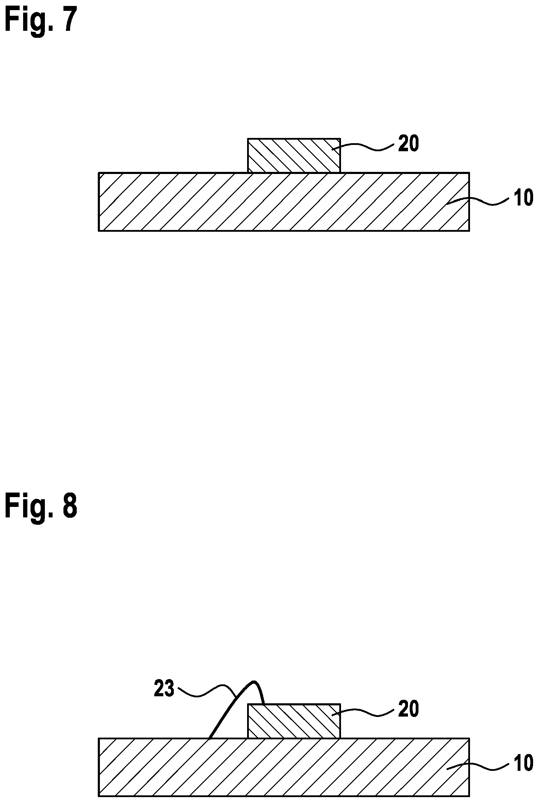

Die Fign. 7 bis 10 zeigen schematisch und beispielhaft ein Verfahren zur Herstellung einer Druckerfassungseinrichtung 1 für eine Messvorrichtung 100 zum Messen eines Druckzustandswerts einer Pflanzenprobe P. Das Verfahren eignet sich insbesondere zur Herstellung der voranstehend beschriebenen Druckerfassungseinrichtung 1 und wird nachfolgend unter Bezugnahme auf die oben beschriebene Druckerfassungseinrichtung 1 erläutert. Die im Zusammenhang mit der Druckerfassungseinrichtung 1 beschriebenen Merkmale und Vorteile gelten somit in analoger Weise auch für das Verfahren und umgekehrt.

Wie in Fig. 7 dargestellt, erfolgt zunächst ein Montieren der Sensoreinrichtung 20 an dem Trägersubstrat 10. Beispielsweise kann die Sensoreinrichtung 20 an die Montageoberfläche 10a des Trägersubstrats 10 angeklebt, angelötet oder auf andere Weise an dieser befestigt werden. Das Montieren der Sensoreinrichtung 20 kann ein Kontaktieren der Sensoreinrichtung 20 bereits beinhalten, z.B., wenn diese wie in Fig. 5 gezeigt aufgebaut ist und mit dem Trägersubstrat 10 verlötet wird. Alternativ erfolgt das Kontaktieren nach dem Befestigen, wie dies beispielhaft in Fig. 8 dargestellt ist, wobei die Sensoreinrichtung 20 beispielsweise über eine Verdrahtung 23 elektrisch kontaktiert wird, z.B. mit an dem Trägersubstrat 10 vorgesehenen Anschlussstrukturen (nicht dargestellt).

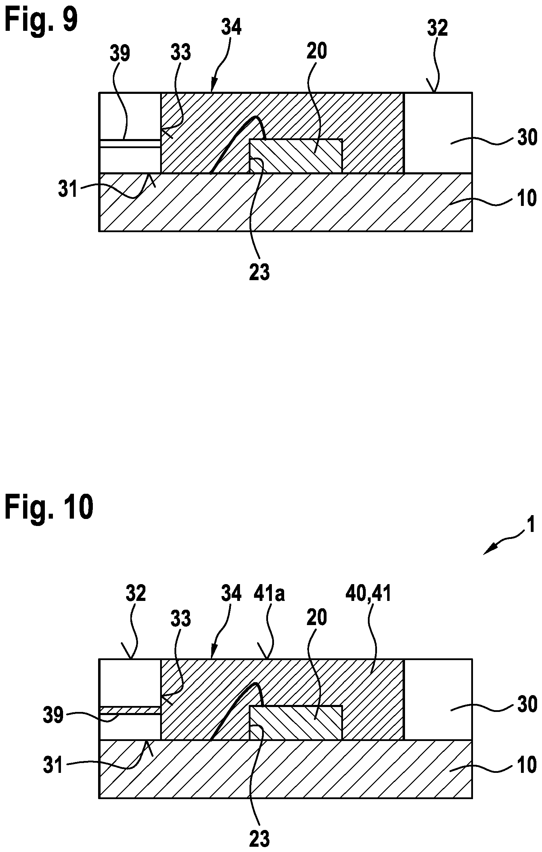

Nach dem Montieren der Sensoreinrichtung 20 erfolgt ein Befestigen des Rahmens 30 an dem Trägersubstrat 10, wie dies in Fig. 9 beispielhaft dargestellt ist. Hierbei wird der Rahmen 30 mit der Befestigungsfläche 31 auf die Montageoberfläche 10a des Trägersubstrats 10 aufgesetzt und derart auf der Montageoberfläche 10a des Trägersubstrats 10 positioniert, dass die Innenfläche 33 die Sensoreinrichtung 20 umschließt. Somit werden der Rahmen 30 und die Sensoreinrichtung separat voneinander an dem Trägersubstrat 10 montiert. Insbesondere wird zuerst die Sensoreinrichtung 20 befestigt und elektrisch kontaktiert und anschließend der Rahmen 30 aufgesetzt. Dadurch kann die Öffnung 34 des Rahmens 30 relativ klein ausfallen, da kein Montageraum zum Einführen von die Sensoreinrichtung 20 haltenden Montagevorrichtungen vorgesehen werden muss. Dies erleichtert die Automatisierung des Verfahrens. Gleichzeitig kann ein kompakterer Aufbau der Druckerfassungseinrichtung 1 realisiert werden.

Allgemein kann der Rahmen 30 stoffschlüssig, beispielsweise durch Ankleben, oder formschlüssig, beispielsweise durch eine Clip-Verbindung, an dem Trägersubstrat 10 befestigt werden.

Anschließend erfolgt ein Füllen der Öffnung 34 des Rahmens 30 mit dem Füllmaterial 40 zur Ausbildung der elastischen Druckkopplungslage 41. Dieser Schritt ist schematisch in Fig. 10 dargestellt. Das Füllmaterial 40 wird hierbei in einem fließfähigen Zustand in die Öffnung 34 des Rahmens 30 eingeführt. Nach dem Einführen erfolgt ein Härten des Füllmaterials 41 in der Öffnung 34, z.B. durch eine chemische Vernetzungsreaktion. Hierzu kann beispielsweise vorgesehen sein, dass das Füllmaterial 41 nach dem Einführen in die Öffnung 34 auf eine bestimmte Temperatur gebracht wird oder mit UV-Licht bestrahlt wird, um die chemische Reaktion auszulösen und/oder abzubrechen.

Das Füllmaterial 40 kann insbesondere direkt in die Öffnung 34 eingefüllt werden, also von einer durch die Kontaktfläche 32 des Rahmens 30 definierten Vorderseite her. Um Lufteinschlüssen vorzubeugen, kann der Rahmen 30 optionale Belüftungsstrukturen aufweisen, z.B. in Form eines sich von der Innenfläche 33 zu der Außenumfangsfläche 35 erstreckenden Entlüftungskanals 39, wie in den Fign. 9 und 10 schematisch dargestellt, und/oder in Form einer Entlüftungsnut 38 wie in Fig 6. schematisch dargestellt, welche an der Innenfläche 33 ausgebildet ist und sich bis zu der Kontaktfläche 31 des Rahmens 30 erstreckt.

Obwohl die vorliegende Erfindung anhand bevorzugter Ausführungsbeispiele vorstehend vollständig beschrieben wurde, ist sie darauf nicht beschränkt, sondern auf vielfältige Art und Weise innerhalb des Anwendungsbereichs der beigefügten Ansprüche modifizierbar.

## Patentansprüche

1. Verfahren zur Herstellung einer Druckerfassungseinrichtung (1) für eine Messvorrichtung (100) zum Messen eines Druckzustandswerts einer Pflanzenprobe (P) mit folgenden Schritten:
Montieren einer Sensoreinrichtung (20) zum Erfassen des Druckzustandswerts an einer Montageoberfläche (10a) eines Trägersubstrats (10);
Befestigen eines Rahmens (30) an der Montageoberfläche (10a) des Trägersubstrats (10), wobei der Rahmen (30) eine Befestigungsfläche (31), eine entgegengesetzt zu der Befestigungsfläche (31) orientierte Kontaktfläche (32) und eine Öffnung (34) definierende und sich zwischen der Befestigungsfläche (31) und der Kontaktfläche (32) erstreckende Innenfläche (33) aufweist, wobei der Rahmen (30) derart an dem Trägersubstrat (10) angeordnet wird, dass die Befestigungsfläche (31) dem Trägersubstrat (10) zugewandt ist und die Innenfläche (33) die Sensoreinrichtung (20) umschließt; und
Füllen der Öffnung (34) des Rahmens (30) mit einem Füllmaterial (40) zur Ausbildung einer elastischen Druckkopplungslage (41).

2. Verfahren nach Anspruch 1, wobei das Montieren der Sensoreinrichtung (20) ein Befestigen der Sensoreinrichtung (20) an dem Trägersubstrat (10) und ein elektrisches Kontaktieren der Sensoreinrichtung (20) umfasst.

3. Verfahren nach Anspruch 1 oder 2, wobei das Füllmaterial (40) in einem fließfähigen Zustand in die Öffnung (34) des Rahmens (30) eingeführt und in der Öffnung (34) gehärtet wird.

4. Verfahren nach einem der voranstehenden Ansprüche, wobei das Füllmaterial (40) von einer durch die Kontaktfläche (32) des Rahmens (30) definierten Vorderseite in die Öffnung (34) des Rahmens (30) eingeführt wird.

5. Verfahren nach einem der voranstehenden Ansprüche, wobei der Rahmen (30) stoffschlüssig, insbesondere durch Ankleben, oder formschlüssig, insbesondere durch eine Clip-Verbindung, an der Montageoberfläche (10a) des Trägersubstrats (10) befestigt wird.

6. Druckerfassungseinrichtung (1) für eine Messvorrichtung (100) zum Messen eines Druckzustandswerts einer Pflanzenprobe (P), mit:
einem Trägersubstrat (10) mit einer Montageoberfläche (10a);
einer an der Montageoberfläche (10a) des Trägersubstrats (10) angeordneten Sensoreinrichtung (20) zum Erfassen des Druckzustandswerts;
einem an der Montageoberfläche (10a) des Trägersubstrat (10) befestigten Rahmen (30), welcher eine dem Trägersubstrat (10) zugewandte Befestigungsfläche (31), eine entgegengesetzt zu der Befestigungsfläche (31) orientierte Kontaktfläche (32) zur Anlage an die Pflanzenprobe (P) und eine Öffnung (34) definierende und sich zwischen der Befestigungsfläche (31) und der Kontaktfläche (32) erstreckende Innenfläche (33) aufweist, wobei die Innenfläche (33) die Sensoreinrichtung (20) umschließt; und
eine die Öffnung (34) füllende elastische Druckkopplungslage (41) zur Anlage an die Pflanzenprobe (P) zum Übertragen einer Kraft an die Sensoreinrichtung (20).

7. Druckerfassungseinrichtung (1) nach Anspruch 6, wobei die Innenfläche (33) im Bereich der Kontaktfläche (32) des Rahmens (30) eine erste Querschnittsfläche der Öffnung (34) und im Bereich der Befestigungsfläche (31) des Rahmens (30) eine zweite Querschnittsfläche der Öffnung (34) definiert, wobei die zweite Querschnittsfläche größer als die erste Querschnittsfläche der Öffnung (34) ist.

8. Druckerfassungseinrichtung (1) nach Anspruch 6 oder 7, wobei das Trägersubstrat (10) durch eine Leiterplatte gebildet ist.

9. Druckerfassungseinrichtung (1) nach einem der Ansprüche 6 bis 8, wobei die Sensoreinrichtung (20) ein Sensorelement (21) zur Erfassung des Druckzustandswerts und zur Erzeugung eines den Druckzustandswert repräsentierenden Drucksignals in Form einer MEMS-Struktur und eine Ausleseeinheit (22) in Form eines ASIC zur Verarbeitung des vom Sensorelement erzeugbaren Drucksignals aufweist.

10. Druckerfassungseinrichtung (1) nach einem der Ansprüche 6 bis 9, wobei der Rahmen (30) ein magnetisches oder magnetisierbares Material aufweist.

11. Druckerfassungseinrichtung (1) nach einem der Ansprüche 6 bis 10, wobei der Rahmen (30) aus einem Kunststoffmaterial, insbesondere aus einem Thermoplastmaterial, einem Duroplastmaterial oder einem Elastomermaterial gebildet ist.

## Claims

1. Method for producing a pressure detection device (1) for a measuring instrument (100) for measuring a pressure state value of a plant specimen (P), comprising the following steps:
mounting a sensor device (20) for detecting the pressure state value on a mounting surface (10a) of a carrier substrate (10);
fastening a frame (30) to the mounting surface (10a) of the carrier substrate (10), the frame (30) having a fastening face (31), a contact face (32) oriented in the opposite direction to the fastening face (31), and an inner face (33) which defines an opening (34) and extends between the fastening face (31) and the contact face (32), the frame (30) being arranged on the carrier substrate (10) in such a way that the fastening face (31) faces the carrier substrate (10) and the inner face (33) surrounds the sensor device (20); and
filling the opening (34) of the frame (30) with a filler material (40) to form an elastic pressure-coupling layer (41) .

2. Method according to Claim 1, wherein mounting the sensor device (20) comprises fastening the sensor device (20) to the carrier substrate (10) and electrically contacting the sensor device (20).

3. Method according to Claim 1 or 2, wherein the filler material (40) is introduced into the opening (34) of the frame (30) in a flowable state and is cured in the opening (34) .

4. Method according to one of the preceding claims, wherein the filler material (40) is introduced into the opening (34) of the frame (30) from a front side defined by the contact face (32) of the frame (30).

5. Method according to one of the preceding claims, wherein the frame (30) is fastened to the mounting surface (10a) of the carrier substrate (10) integrally, in particular by adhesive bonding, or in an interlocking manner, in particular by a clip connection.

6. Pressure detection device (1) for a measuring instrument (100) for measuring a pressure state value of a plant specimen (P), comprising:
a carrier substrate (10) with a mounting surface (10a);
a sensor device (20), arranged on the mounting surface (10a) of the carrier substrate (10), for detecting the pressure state value;
a frame (30) which is fastened to the mounting surface (10a) of the carrier substrate (10) and has a fastening face (31) facing the carrier substrate (10), a contact face (32), oriented in the opposite direction to the fastening face (31), for making contact with the plant specimen (P), and an inner face (33) which defines an opening (34) and extends between the fastening face (31) and the contact face (32), the inner face (33) surrounding the sensor device (20); and
an elastic pressure-coupling layer (41), which fills the opening (34), for making contact with the plant specimen (P) for transmitting a force to the sensor device (20).

7. Pressure detection device (1) according to Claim 6, wherein the inner face (33), in the region of the contact face (32) of the frame (30), defines a first cross-sectional area of the opening (34) and, in the region of the fastening face (31) of the frame (30), a second cross-sectional area of the opening (34), wherein the second cross-sectional area is larger than the first cross-sectional area of the opening (34).

8. Pressure detection device (1) according to Claim 6 or 7, wherein the carrier substrate (10) is formed by a printed circuit board.

9. Pressure detection device (1) according to one of Claims 6 to 8, wherein the sensor device (20) has a sensor element (21) for detecting the pressure state value and for generating a pressure signal, which represents the pressure state value, in the form of an MEMS structure and a reading unit (22) in the form of an ASIC for processing the pressure signal that can be generated by the sensor element.

10. Pressure detection device (1) according to one of Claims 6 to 9, wherein the frame (30) contains a magnetic or magnetizable material.

11. Pressure detection device (1) according to one of Claims 6 to 10, wherein the frame (30) is formed from a plastic material, in particular from a thermoplastic material, a thermoset material or an elastomeric material.

## Revendications

1. Procédé permettant de fabriquer un dispositif de détection de pression (1) pour un dispositif de mesure (100) servant à mesurer une valeur d'état de pression d'un échantillon végétal (P), comprenant les étapes suivantes consistant à :
monter un dispositif capteur (20) servant à détecter la valeur d'état de pression sur une surface de montage (10a) d'un substrat porteur (10) ;
fixer un cadre (30) à la surface de montage (10a) du substrat porteur (10), dans lequel le cadre (30) présente une surface de fixation (31), une surface de contact (32) orientée à l'opposé de la surface de fixation (31) et une surface intérieure (33) définissant une ouverture (34) et s'étendant entre la surface de fixation (31) et la surface de contact (32), dans lequel le cadre (30) est disposé sur le substrat porteur (10) de telle sorte que la surface de fixation (31) est tournée vers le substrat porteur (10) et la surface intérieure (33) entoure le dispositif capteur (20) ; et
remplir l'ouverture (34) du cadre (30) avec un matériau de remplissage (40) en réalisant une couche de couplage de pression élastique (41).

2. Procédé selon la revendication 1, dans lequel le montage du dispositif capteur (20) comprend une fixation du dispositif capteur (20) au substrat porteur (10) et une mise en contact électrique du dispositif capteur (20).

3. Procédé selon la revendication 1 ou 2, dans lequel le matériau de remplissage (40) dans un état fluide est introduit dans l'ouverture (34) du cadre (30) et est durci dans l'ouverture (34).

4. Procédé selon l'une quelconque des revendications précédentes, dans lequel le matériau de remplissage (40) est introduit dans l'ouverture (34) du cadre (30) à partir d'une face frontale définie par la surface de contact (32) du cadre (30).

5. Procédé selon l'une quelconque des revendications précédentes, dans lequel le cadre (30) est fixé à la surface de montage (10a) du substrat porteur (10) par liaison de matière, en particulier par collage, ou par complémentarité de forme, en particulier par un assemblage à encliquetage.

6. Dispositif de détection de pression (1) destiné à un dispositif de mesure (100) servant à mesurer une valeur d'état de pression d'un échantillon végétal (P), comprenant :
un substrat porteur (10) pourvu d'une surface de montage (10a) ;
un dispositif capteur (20), disposé sur la surface de montage (10a) du substrat porteur (10), pour détecter la valeur d'état de pression ;
un cadre (30) fixé à la surface de montage (10a) du substrat porteur (10) et qui présente une surface de fixation (31) tournée vers le substrat porteur (10), une surface de contact (32) orientée à l'opposé de la surface de fixation (31) pour être adjacente à l'échantillon végétal (P), et une surface intérieure (33) définissant une ouverture (34) et s'étendant entre la surface de fixation (31) et la surface de contact (32), dans lequel la surface intérieure (33) entoure le dispositif capteur (20) ; et
une couche de couplage de pression élastique (41) remplissant l'ouverture (34), pour être adjacente à l'échantillon végétal (P) afin de transmettre une force au dispositif capteur (20).

7. Dispositif de détection de pression (1) selon la revendication 6, dans lequel la surface intérieure (33) définit au niveau de la surface de contact (32) du cadre (30) une première superficie de la section de l'ouverture (34) et au niveau de la surface de fixation (31) du cadre (30) une deuxième superficie de la section de l'ouverture (34), dans lequel la deuxième superficie de la section est supérieure à la première superficie de la section de l'ouverture (34).

8. Dispositif de détection de pression (1) selon la revendication 6 ou 7, dans lequel le substrat porteur (10) est formé par une carte de circuits imprimés.

9. Dispositif de détection de pression (1) selon l'une quelconque des revendications 6 à 8, dans lequel le dispositif capteur (20) présente un élément capteur (21) pour détecter la valeur d'état de pression et pour générer un signal de pression représentant la valeur d'état de pression, sous la forme d'une structure MEMS et une unité de lecture (22) sous la forme d'un ASIC pour traiter le signal de pression pouvant être généré par l'élément capteur.

10. Dispositif de détection de pression (1) selon l'une quelconque des revendications 6 à 9, dans lequel le cadre (30) présente un matériau magnétique ou magnétisable.

11. Dispositif de détection de pression (1) selon l'une quelconque des revendications 6 à 10, dans lequel le cadre (30) est formé d'une matière plastique, en particulier d'une matière thermoplastique, d'une matière plastique thermodurcissable ou d'une matière élastomère.
